# FASCICULE DE BREVET EUROPEEN

(11) **EP 3 740 153 B1**
(45) Date de publication et mention de la délivrance du brevet: **06.09.2023**
(21) Numéro de dépôt: 19705554.4
(22) Date de dépôt: 16.01.2019
(51) Int. Cl.: A61C 5/00, A61C 7/00, A61C 7/08, A61K 6/00

(54) **PROCÉDÉ DE RÉALISATION D'UN DISPOSITIF DE CONTENTION COMPORTANT UN APPAREIL DE CONTENTION ET UNE GOUTTIÈRE DE POSITIONNEMENT, ET DISPOSITIF DE CONTENTION OBTENU**
VERFAHREN ZUR KONSTRUKTION EINER EINSCHLUSSVORRICHICHTUNG MIT EINER EINSCHLUSSVORRICHTUNG UND EINEM POSITIONIERUNGSKANAL SOWIE ERHALTENE EINSCHLUSSVORRICHTUNG
METHOD FOR CONSTRUCTING A CONTAINMENT DEVICE COMPRISING A CONTAINMENT DEVICE AND A POSITIONING DUCT, AND DEVICE FOR CONTAINMENT OBTAINED

(30) Priorité: 19.01.2018 FR 1850434
(43) Date de publication de la demande: 25.11.2020
(73) Titulaire: BIOTECH DENTAL, 13300 Salon-de-Provence (FR)
(72) Inventeur: FOREST, Alexandre, 30127 Bellegarde (FR); SIREIX, Christophe, 74250 Bogeve (FR)
(74) Mandataire: Santarelli
(86) Numéro de dépôt international: PCT/FR2019/050091
(87) Numéro de publication internationale: WO 2019/141943

(56) Documents cités:
- WO-A1-2017/207967
- CN-A- 101 559 006
- US-A1- 2012 028 221
- ARLEN J. HURT: "Post-Treatment Fixed Lingual Retainers", THE AMERICAN ACADEMY FOR CLEAR ALIGNER THERAPY JOURNAL, vol. 1, no. 1, 30 novembre 2012 (2012-11-30), pages 22-26, XP055394047,

## Description

La présente invention concerne un procédé de réalisation d'un dispositif de contention comportant un appareil de contention et une gouttière de positionnement.

Elle concerne aussi un dispositif de contention obtenu par un tel procédé.

Dans le domaine de l'orthodontie, les plans de traitement actifs, c'est-à-dire pour corriger une mauvaise posture des dents, dans un but fonctionnel ou esthétique, s'établissent à l'aide de différents moyens, par exemple tels que :
- les appareils fixes, qui se collent sur les dents par des bagues (en métal, céramique ou élastomère) et qui sont reliées les unes aux autres par un câble, ou tige de métal par exemple ; ou
- les appareils amovibles, souvent transparents, sous forme de gouttière, réalisés en matière plastique.

Une fois l'appareil orthodontique actif posé, il est nécessaire de maintenir les résultats acquis par un appareil de contention sous peine de récidive de l'anomalie initiale ou d'apparition d'une nouvelle pathologie.

Cet appareil de contention peut aussi être fixe ou amovible.

S'il est amovible, il sera porté selon une prescription, en permanence (i.e. 24h/24), ou une partie du temps (par exemple 15h/24 ou la nuit ou 8h/24h). La durée du port est au minimum d'un an. Il est parfois nécessaire de le garder 5 ans, 10 ans ou toute la vie. Dans ce cas, il s'agit par exemple d'une plaque palatine simple, ou d'une plaque de Hawley, ou d'activateurs, ou de gouttières, c'est-à-dire une sorte de protège-dents qui peut être réalisé en résine molle ou en polymère transparent par exemple.

S'il est fixe, il s'agit bien souvent d'un appareil de contention, polymérisé ou collé dans la bouche d'un patient, qui stabilise les résultats acquis. Dans ce cas, il peut s'agir, par exemple, d'un fil métallique torsadé collé directement sur chacune des dents qu'il recouvre, ou d'un fil métallique collé sur deux facettes métalliques elles-mêmes collées directement sur deux dents, par exemple les canines, ou encore d'un arc lingual sur barre, c'est-à-dire un fil métallique directement soudé à des bagues canines ou prémolaire.

En outre, un appareil de contention chez l'adulte est strictement individualisé.

Cependant, de tels appareils de contention ont plusieurs limites, par exemple :
- Ceux qui sont en métal ont une couleur grise, terne qui se transmet éventuellement au dents et qui nuit à leur aspect iridescent.
- Les contentions métalliques sont trop rigides et engendre par exemple :
   o Des phénomènes de décollement, et/ou
   o Une immobilisation des dents qui induit une perte osseuse et potentiellement une perte des dents.
- Les fils métalliques peuvent rayer les dents et/ou induire des caries et/ou des risques de fissure nuisant à la résilience de celles-ci.
- Ceux qui sont polymérisés dans la bouche d'un patient demandent un matériel conséquent spécifique au praticien. De plus, la polymérisation en bouche est difficile à contrôler. Bien souvent les couches sont trop épaisses pour permettre une bonne polymérisation. La polymérisation sous la fibre est très difficile, voire impossible dans certains cas. Le polissage est compliqué. L'épaisseur de l'appareil de contention est difficile, voire impossible, à contrôler. Du matériau s'immisce entre des dents, ce qui est très difficile, voire impossible, à nettoyer.

Le document US2012/028221 A1 divulgue un procédé de réalisation d'un appareil de contention comportant une étape de modélisation d'un composant extracoronaire monobloc et une étape de réalisation du composant.

L'objet de la présente demande vise à améliorer au moins en partie les inconvénients précités, menant en outre à d'autres avantages.

A cet effet, est proposé un procédé de réalisation d'un dispositif de contention comportant au moins les étapes suivantes :
- Une étape de modélisation d'un appareil de contention sous forme de ruban ;
- Une étape de réalisation de l'appareil de contention par usinage d'une galette de matériau polymère composite ou par impression tridimensionnelle ;
- Une étape de réalisation d'une gouttière de positionnement par thermoformage sur un ensemble-support qui comporte l'appareil de contention et une matrice, représentant au moins une partie d'arcade dentaire, sur laquelle l'appareil de contention est positionné ;
- Une étape d'obtention du dispositif de contention sur la matrice, le dispositif de contention comportant la gouttière de positionnement et l'appareil de contention sous forme de ruban positionné dans la gouttière de positionnement ; et
- Une étape de séparation du dispositif de contention par rapport à la matrice.

Grâce à un tel procédé, il est ainsi possible d'obtenir un appareil de contention de type « fixe » (car il sera ensuite fixe dans la bouche du patient), préalablement réalisé, et qui est facile à mettre en place pour être ensuite collé sur des dents d'un patient.

Une telle gouttière de positionnement est ainsi configurée pour contenir l'appareil de contention sous forme de ruban et ultérieurement le positionner, i.e. le transférer, dans la bouche d'un patient, à l'emplacement désiré.

De plus, la souplesse relative du matériau de l'appareil de contention permet notamment de réaliser une contention monobloc, par exemple de prémolaire à prémolaire, ce qui serait impossible à réaliser en métal car un tel appareil de contention induirait alors trop de contraintes sur les dents.

Un tel appareil de contention est en outre très discret, voire invisible, et il est à la fois suffisamment rigide de manière à brider les dents mais avec une souplesse relative, ce qui permet notamment de le rendre bien moins traumatisant pour les dents ou les tissus et/ou moins contraignant pour son porteur.

Dans un exemple de mise en oeuvre, le procédé comporte en outre une étape de modélisation d'au moins une barre de positionnement, ou ergot, de préférence d'une paire de barres de positionnement, ou d'ergots, sur l'appareil de contention.

Une barre de positionnement, ou ergot, est ici un élément configuré pour maintenir l'appareil de contention en place dans la gouttière de positionnement. Il s'agit d'une extrusion, ou d'une paire d'extrusions, solidaire de l'appareil de contention, par exemple en étant réalisée conjointement à l'appareil de contention. Un tel ergot est de préférence formé à une extrémité du ruban formant l'appareil de contention.

Le procédé comporte alors en outre une étape de réalisation de l'au moins un ergot, de préférence d'une paire d'ergots ; par exemple par impression 3D ou usinage.

De préférence, l'étape de réalisation de l'au moins un ergot, ou de la paire d'ergots, est alors réalisée au cours de l'étape de réalisation de l'appareil de contention. Autrement dit, l'étape de réalisation de l'appareil de contention comporte au moins une étape de réalisation de l'au moins un ergot.

Ainsi, l'appareil de contention et l'au moins un ergot sont réalisés d'un seul tenant.

Dans un autre exemple de mise en oeuvre, le procédé comporte en outre une étape de modélisation d'au moins un connecteur sur l'appareil de contention, de préférence d'une paire de connecteurs.

Un connecteur est ici un élément configuré pour maintenir en place l'appareil de contention durant son usinage lorsqu'il est fabriqué par usinage. Un connecteur demeure connecté à la fois à l'appareil de contention et au reste de la galette de matériau dans laquelle l'appareil de contention est usiné, tant que l'étape d'usinage n'est pas terminée. Autrement dit, un connecteur forme un pont, un lien, entre un appareil de contention et la galette de matériau dans laquelle il est usiné.

Le procédé comporte alors en outre une étape de réalisation de l'au moins un connecteur sur l'appareil de contention, de préférence d'une paire de connecteurs.

Autrement dit, le cas échéant, l'étape de réalisation de l'appareil de contention comporte aussi au moins une étape de réalisation d'au moins un ergot et/ou d'au moins un connecteur sur l'appareil de contention.

L'éventuelle présence d'un ergot est tout à fait indépendante de celle d'un connecteur, et inversement.

L'étape de réalisation de l'au moins un connecteur, ou de la paire de connecteurs, sur l'appareil de contention comporte par exemple une étape d'usinage de la galette de matériau.

Par exemple, l'étape d'usinage de la galette de matériau de l'étape de réalisation de l'appareil de contention comporte l'étape d'usinage de la galette de matériau de l'étape de réalisation de l'au moins un connecteur.

Par exemple, l'étape de réalisation de l'appareil de contention avec l'au moins un connecteur comporte une étape d'usinage de la même galette dudit matériau.

Ou par exemple, l'étape de réalisation de l'appareil de contention, avec l'au moins un ergot et/ou l'au moins un connecteur, comporte une étape d'usinage de la même galette dudit matériau.

Selon un exemple de mise en oeuvre privilégié, la galette de matériau polymère composite comporte un matériau polymère composite amorphe à réticulation croisée.

Un tel matériau est par exemple commercialisé sous la référence Bredent HIPC^{®} (High Impact Polymer Composite).

Alors qu'un tel matériau était connu pour d'autres applications, il s'est avéré particulièrement intéressant pour la réalisation d'appareil de contention de type barre, en particulier de par sa couleur proche de celle de l'émail des dents et par ses propriétés mécaniques, et en particulier pour des sollicitations en flexion.

Dans un exemple particulier, la galette de matériau polymère composite comporte un matériau polymère composite amorphe à réticulation croisée avec une base de méthacrylate.

Le méthacrylate est ici un mélange de monomères liquides dont la réticulation est initiée par un peroxyde. Le durcissement du mélange de monomères, en fonction du taux de peroxyde, s'effectue soit à température ambiante soit à basse température, voire à température négative. Les mélanges réticulés se caractérisent notamment par :
- Une mise en service très rapide ;
- Un durcissement possible à basse température, voire à température négative ;
- Une bonne résistance mécanique et chimique ;
- Une flexibilité plus ou moins importante ;
- Une forte adhérence sur de nombreux supports.

Dans un cas de réalisation de l'appareil de contention par impression tridimensionnelle, des matériaux intéressants à utiliser sont par exemple le matériau commercialisé sous la référence « NextDent C&B »^{®} ou le matériau commercialisé sous la référence « NextDent C&B MFH (Micro Filled Hybrid) »^{®}, tous deux de Nextdent^{®}.

Dans un exemple de mise en oeuvre particulier, l'étape de réalisation de la gouttière de positionnement par thermoformage comporte une étape de thermoformage d'un film de matériau comportant du néoprène ou un élastomère.

Il s'agit par exemple d'un film plastique, en PET, PETG.

Dans un autre exemple de mise en oeuvre particulier, l'étape de réalisation de la gouttière de positionnement par thermoformage comporte une étape de thermoformage d'un film de matériau avec une épaisseur moyenne comprise entre 1,5 mm et 2,5 mm, et de préférence entre 1,7 mm et 2,3 mm.

On entend ici par film une feuille de matériau ayant une épaisseur au maximum égale à 5 mm, voire 4 mm. De préférence, son épaisseur minimale est d'au moins 0,1 mm, ou mieux d'au moins 0,5 mm.

Dans un exemple de mise en oeuvre privilégié, le procédé comporte une étape de détourage, c'est-à-dire de fraisage, du film de matériau à mi-hauteur de l'arcade dentaire de la matrice.

Autrement dit, le film est détouré au niveau de la mi-hauteur des dents pour un meilleur positionnement de la gouttière dans la bouche d'un patient pour y transférer l'appareil de contention.

Ainsi, un film de matériau est apposé sur l'ensemble-support et est chauffé.

Il est ensuite, par exemple, détouré pour ne couvrir qu'une mi-hauteur des dents par exemple, tout en englobant, au moins en partie, l'appareil de contention.

Il forme ainsi une gouttière dans laquelle l'appareil de contention est positionné.

Autrement dit, la gouttière de positionnement est d'une certaine manière surmoulée sur au moins l'appareil de contention.

Dans un exemple de mise en oeuvre, le procédé comporte en outre une étape de prise d'empreinte d'au moins la partie d'une arcade dentaire à représenter par la matrice.

Par exemple, l'étape de prise d'empreinte comporte une étape de scan intra-buccal.

Selon une option intéressante, le procédé comporte en outre une étape de polissage de l'appareil de contention.

Selon une option intéressante, le procédé comporte en outre une étape de nettoyage de l'appareil de contention.

Selon un exemple, le procédé comporte en outre une étape de suppression de l'au moins un ergot, ou de la paire d'ergots.

Selon un autre exemple, le procédé comporte en outre une étape de suppression de l'au moins un connecteur, ou de la paire de connecteurs. Cette étape a avantageusement lieu à la fin de l'étape d'usinage de l'appareil de contention. L'au moins un connecteur est par exemple retiré par fraisage.

Est également proposé, selon un aspect de l'invention non revendiqué, un dispositif de contention obtenu par un procédé comportant au moins une partie des caractéristiques susmentionnées, le dispositif de contention comportant une gouttière de positionnement et un appareil de contention sous forme de ruban positionné dans la gouttière de positionnement.

De préférence, l'appareil de contention présente une épaisseur moyenne d'au moins 0,3 mm, par exemple comprise entre 0,3 mm et 1 mm. Son épaisseur maximale est par exemple au maximum égale à 3 mm.

De préférence, la gouttière de positionnement comporte une épaisseur moyenne comprise entre 1,5 mm et 2,5 mm, et de préférence entre 1,7 mm et 2,3 mm. Son épaisseur maximale est par exemple au maximum égale à 5 mm.

L'invention, selon un exemple de réalisation, sera bien comprise et ses avantages apparaîtront mieux à la lecture de la description détaillée qui suit, donnée à titre indicatif et nullement limitatif, en référence aux dessins annexés dans lesquels :
La figure 1 présente un exemple de modélisation numérique d'une prise d'empreinte intra-buccale,
Les figures 2a et 2b présentent un exemple de modélisation de l'appareil de contention sur l'arcade dentaire respectivement sans et avec ergots,
La figure 3 présente un exemple de modélisation d'appareils de contention dans une galette de matériau selon un exemple de mise en oeuvre,
La figure 4 présente un exemple d'usinage d'appareils de contention dans une galette de matériau selon un exemple de mise en oeuvre, et
La figure 5 illustre un exemple de réalisation d'une gouttière de positionnement sur un ensemble-support qui comporte un appareil de contention et une matrice représentant au moins une partie d'arcade dentaire sur laquelle l'appareil de contention est positionné.

Les éléments identiques représentés sur les figures précitées sont identifiés par des références numériques identiques.

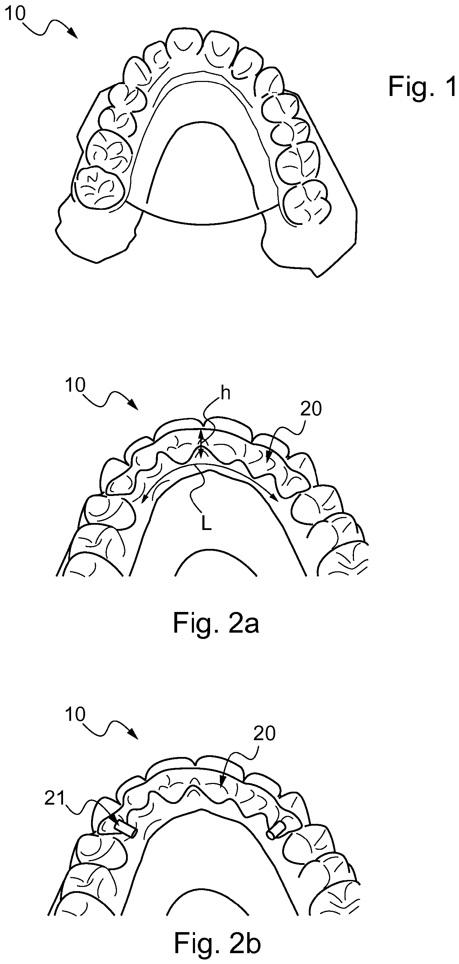

Une prise d'empreinte d'au moins une partie d'une arcade dentaire 10 d'un patient est réalisée. Au moins un fichier numérique permettant une modélisation 3D, par exemple tel que représentée en figure 1, est obtenu.

Un tel fichier est par exemple extrait par l'entremise d'un scan tridimensionnel par exemple (par exemple du type Imetrics^{®}). Il est aussi possible de prendre directement une empreinte en bouche grâce à un scan intra-buccal, par exemple par une caméra CONDOR^{®}.

Après cela, le fichier 3D de l'empreinte est repris sur un logiciel (tel que par exemple Dental-Wings^{®}). L'orientation des arcades est possiblement optimisée afin de réduire des contre-dépouilles sur des faces vestibulaires des dents par rapport à un axe vertical théorique ; autrement dit, cette opération permet « d'ouvrir » la face interne des dents, en particulier où un appareil de contention selon un exemple de mise en oeuvre de l'invention sera positionné.

Une modélisation de l'appareil de contention 20 peut ensuite être réalisée.

L'appareil de contention 20 est modélisé en fonction du modèle numérique de la partie d'arcade dentaire 10, de préférence sous forme d'un ruban, éventuellement en prenant soin de préserver une épaisseur minimum de 0,3 mm de préférence.

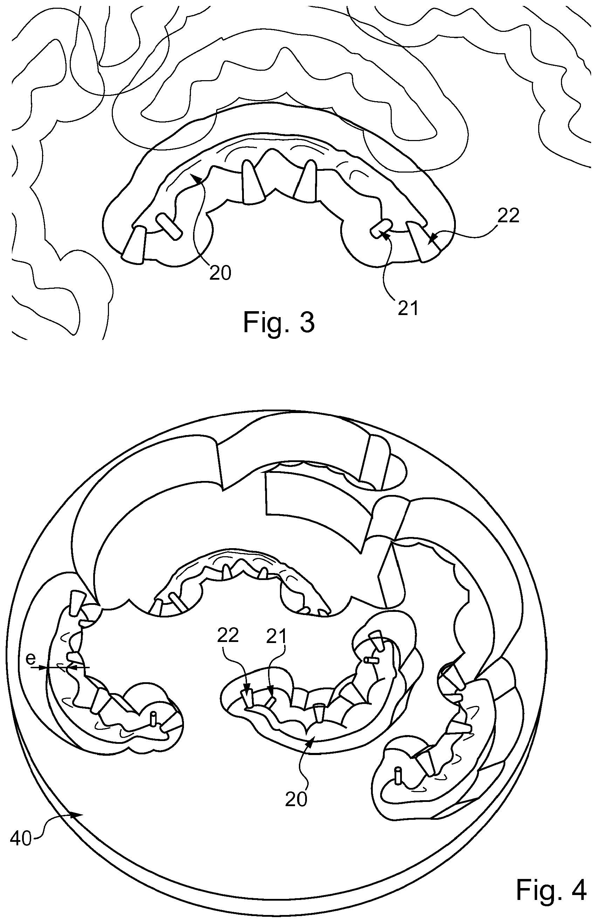

L'appareil de contention 20 est ici dans la mesure du possible un ruban. Un ruban désigne ici une barre dont une dimension, par exemple une épaisseur e parmi une hauteur h, une longueur L (représentées figure 2a) et une épaisseur e (représentée figure 4) de l'appareil, est bien inférieure aux deux autres dimensions, par exemple à la longueur L et à la hauteur h. Ainsi, ici, la longueur L correspond à la dimension de l'appareil selon l'arcade dentaire et correspond par exemple au nombre de dents recouvertes par l'appareil tandis que la hauteur h correspond à une dimension transverse selon une hauteur d'une dent ; une épaisseur e correspond ainsi à la troisième dimension. Par comparaison, un appareil sous forme de fil présenterait une hauteur sensiblement égale à son épaisseur.

Dans un exemple de mise en oeuvre, l'appareil de contention est configuré pour couvrir 2, ou 4, ou 6, ou même 8 dents, ou même davantage, en fonction des cas ; il s'agit par exemple de couvrir un secteur d'une canine à l'autre canine, comme illustré sur les figures 2a et 2b, ou encore d'une prémolaire à l'autre prémolaire, d'une même arcade dentaire.

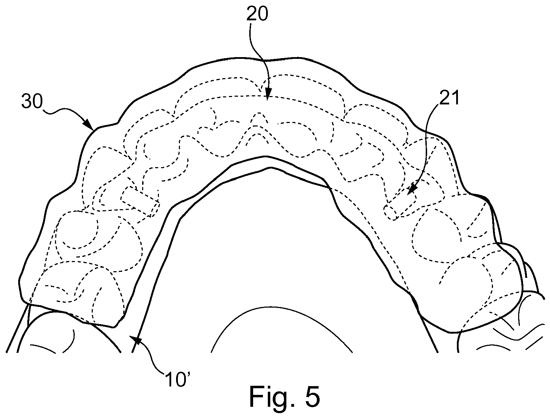

Lors de la modélisation, au moins un ergot 21, configuré pour ultérieurement contribuer au positionnement de l'appareil de contention 20 dans une gouttière de positionnement 30 (représentée figure 5), est ajouté à l'appareil de contention 20, c'est-à-dire au ruban, comme l'illustre par exemple la figure 2b en comparaison avec la figure 2a, lesquelles présentent un exemple de modélisation de l'appareil de contention 20 sur une partie d'arcade dentaire 10. Un tel ergot 21 est un montage temporaire qui sera retiré après apposition de l'appareil de contention dans une bouche.

De préférence, il y a une paire d'ergots, un ergot à chaque extrémité de l'appareil de contention 20 par exemple.

Comme le montrent les figures 3 et 4, au moins un connecteur 22 est aussi éventuellement ajouté dans au moins un des secteurs les plus résistants de l'appareil de contention 20, par exemple afin d'éviter de fragiliser l'appareil de contention durant une étape de réalisation par usinage. De préférence, les connecteurs 22 sont au moins deux, c'est-à-dire une paire, ou trois, ou plus.

Si un appareil de contention 20 est réalisé par impression tridimensionnelle, il est possible de se dispenser de connecteurs 22.

Lors d'une étape de modélisation, plusieurs appareils de contention 20, avec chacun au moins un ergot 21, voire un connecteur 22, sont modélisés et positionnés les uns par rapport aux autres afin d'optimiser leur réalisation simultanément. Par exemple leur usinage dans une même galette 40 ou leur impression tridimensionnelle au cours d'une même fournée.

Comme l'illustre la figure 3, pour la réalisation d'au moins un appareil de contention 20, un fichier de CAO, par exemple un fichier sous format STL, présentant au moins un appareil de contention 20, et de préférence plusieurs, est transféré, par exemple à un logiciel de préparation d'usinage, par exemple le logiciel Work-NC^{®}. Une orientation du modèle est faite de manière à proposer la face linguale ou palatine sans contre dépouille par rapport à un axe d'attaque d'une fraise.

Un tel mode de réalisation est avantageux car il n'y a ainsi pas de discontinuité sur la géométrie de l'appareil de contention qui peut être usiné d'un seul trait, par exemple sans retournement de la galette de matériau dans laquelle l'usinage est réalisé.

Dans un exemple privilégié de mise en oeuvre, l'usinage se fait dans une galette de matériau polymère composite amorphe, à réticulation croisée, ce qui est par exemple illustré figure 4. Un tel matériau est par exemple commercialisé sous la référence Bredent HIPC^{®} (High Impact Polymer Composite)).

Dans un exemple privilégié de mise en oeuvre, l'usinage se fait avec une machine dite « de laboratoire », telle que par exemple commercialisée sous la dénomination Roland DWX 510^{®} ou encore IMES-ICORE CORITEC 350i^{®}.

Une fois l'usinage terminé, l'au moins un connecteur 22 est retiré, par exemple par fraisage.

Dans un exemple de mise en oeuvre, l'appareil de contention 20 une fois fini est ensuite poli et nettoyé.

Il est ensuite assemblé avec une gouttière de positionnement, décrite ci-dessous, et l'ensemble ainsi formé, c'est-à-dire ici un dispositif de contention, est prêt pour être posé in situ, c'est-à-dire pour placer l'appareil de contention dans la bouche d'un patient.

La gouttière de positionnement est configurée pour servir pour une phase de mise en place de l'appareil de contention dans la bouche.

Une telle gouttière de positionnement est parfois également désignée par « transfert ».

En pratique, la gouttière de positionnement a un comportement plutôt souple, par exemple à l'instar d'un élastomère 70 shore, de manière à pouvoir se conformer sur les dents et leurs implantations.

La figure 5 illustre un exemple intéressant de réalisation de la gouttière de positionnement.

Une matrice 10' représentant au moins une partie d'arcade dentaire 10 est réalisée, par exemple par impression 3D ou par moulage.

Par exemple, au moins la matrice 10' est imprimée en résine, par exemple avec une imprimante Stratasys Eden 50^{®}.

Un appareil de contention 20, tel qu'obtenu à l'issue de l'étape de réalisation de l'appareil de contention, est positionné sur la matrice 10' à laquelle il correspond ; autrement dit l'appareil de contention 20 modélisé et réalisé en fonction d'une partie d'arcade dentaire 10 est positionné sur une matrice 10' représentant la même partie d'arcade dentaire 10. L'appareil de contention 20 comporte alors avantageusement au moins un ergot 21 ou une paire d'ergots 21.

L'appareil de contention 20 assemblé en position sur la matrice 10' forme ainsi, avec la matrice 10', un ensemble-support sur lequel la gouttière de positionnement 30 est ensuite réalisée.

Dans un exemple de réalisation, la gouttière de positionnement 30 est réalisée par application d'un film thermoformé sur l'ensemble-support.

Le film est par exemple un film dont l'épaisseur peut varier ainsi que la composition. Il s'agit par exemple d'un film plastique, en PET, PETG ou encore en élastomère ou en néoprène. Son épaisseur moyenne est par exemple comprise entre 1,5 mm et 2,5 mm, et de préférence entre 1,7 mm et 2,3 mm ; et est au maximum égale à 5 mm, voire 4 mm.

Le film est apposé sur l'ensemble support et est chauffé. Il est ensuite détouré pour ne couvrir qu'une mi-hauteur des dents par exemple, tout en englobant au moins une partie de l'appareil de contention. Il forme ainsi une gouttière dans laquelle l'appareil de contention est positionné. Autrement dit, la gouttière de positionnement est d'une certaine manière surmoulée sur au moins l'appareil de contention.

Une fois l'appareil de contention 20 et la gouttière de positionnement 30 réalisés, ils sont détachés de la matrice 10'. Le dispositif de contention est ainsi obtenu.

Il est ensuite possible de facilement transférer l'appareil de contention 20 dans la bouche d'un patient.

Par exemple, la pose s'effectue par un mordançage préalable de la face interne des dents concernées. De la colle est appliquée sur ces dents ; une telle colle est par exemple commercialisée sous la dénomination Panavia V5 KURARAY^{®}. Le dispositif de contention, formé par l'appareil de contention 20 positionné dans la gouttière de positionnement 30, est alors appliqué sur l'arcade dentaire concernée. La colle est photo-polymérisée puis la gouttière de positionnement est retirée. Le cas échéants, l'au moins un ergot est aussi ensuite retiré de l'appareil de contention, par exemple à l'aide d'une fraise, directement dans la bouche.

Un tel appareil de contention peut ensuite être gardé temporairement ou à vie.

Le mimétisme est très bon de par la couleur naturelle du matériau utilisé et la faible épaisseur de l'appareil de contention qui permet encore davantage de s'adapter à la couleur des dents par transparence.

De plus, le matériau peut avoir différentes teintes ce qui permet de s'ajuster au mieux à celle des dents du patient auquel l'appareil de contention est destiné.

Un tel appareil de contention est ainsi bien plus esthétique, résistant et confortable et s'ajuste bien mieux à une arcade dentaire.

## Revendications

1. Procédé de réalisation d'un dispositif de contention (20, 30) comportant au moins les étapes suivantes :
- Une étape de modélisation d'un appareil de contention (20) sous forme de ruban ;
- Une étape de réalisation de l'appareil de contention (20) par usinage d'une galette (40) de matériau polymère composite ou par impression tridimensionnelle ;
- Une étape de réalisation d'une gouttière de positionnement (30) par thermoformage sur un ensemble-support qui comporte l'appareil de contention (20) et une matrice (10'), représentant au moins une partie d'arcade dentaire (10), sur laquelle l'appareil de contention (20) est positionné ;
- Une étape d'obtention du dispositif de contention (20, 30) sur la matrice (10'), le dispositif de contention comportant la gouttière de positionnement (30) et l'appareil de contention (20) sous forme de ruban positionné dans la gouttière de positionnement (30) ; et
- Une étape de séparation du dispositif de contention (20, 30) par rapport à la matrice (10').

2. Procédé selon la revendication 1 dans lequel l'étape de réalisation de la gouttière de positionnement (30) par thermoformage comporte une étape de thermoformage d'un film de matériau comportant du néoprène ou un élastomère, et avec une épaisseur moyenne comprise entre 1,5 mm et 2,5 mm, et de préférence entre 1,7 mm et 2,3 mm.

3. Procédé selon l'une quelconque des revendications 1 ou 2 comportant une étape de modélisation d'au moins un ergot (21) sur l'appareil de contention (20) et dans lequel l'étape de réalisation de l'appareil de contention (20) comporte au moins une étape de réalisation de l'au moins un ergot (21).

4. Procédé selon l'une quelconque des revendications 1 à 3 comportant une étape de modélisation d'au moins un connecteur (22) sur l'appareil de contention (20) et dans lequel l'étape de réalisation de l'appareil de contention (20) avec l'au moins un connecteur (22) comporte une étape d'usinage de la même galette dudit matériau (40).

5. Procédé selon l'une quelconque des revendications 1 à 4 comportant en outre une étape de polissage et/ou une étape de nettoyage de l'appareil de contention (20).

6. Procédé selon l'une quelconque des revendications 1 à 5 dans lequel la galette (40) de matériau polymère composite comporte un matériau polymère composite amorphe à réticulation croisée.

7. Procédé selon l'une quelconque des revendications 1 à 6 comportant en outre une étape de prise d'empreinte d'au moins la partie d'une arcade dentaire (10) à représenter par la matrice (10').

8. Procédé selon la revendication 7 dans lequel l'étape de prise d'empreinte comporte une étape de scan intra-buccal.

## Patentansprüche

1. Verfahren zum Herstellen einer Retainervorrichtung (20, 30), das mindestens die folgenden Schritte umfasst:
- einen Schritt des Modellierens eines Retainers (20) in Form eines Bandes;
- einen Schritt des Herstellens des Retainers (20) durch maschinelle Bearbeitung einer Scheibe (40) aus polymerem Verbundmaterial oder durch dreidimensionalen Druck;
- einen Schritt des Herstellens einer Positionierungsschiene (30) durch Thermoformen auf einer Trägeranordnung, die den Retainer (20) und eine Matrize (10'), die zumindest einen Teil des Zahnbogens (10) darstellt und auf der der Retainer (20) positioniert ist, umfasst;
- einen Schritt des Erhaltens der Retainervorrichtung (20, 30) auf der Matrize (10'), wobei die Retainervorrichtung die Positionierungsschiene (30) und den Retainer (20) in Form eines Bandes, der in der Positionierungsschiene (30) positioniert ist, umfasst; und
- einen Schritt des Trennens der Retainervorrichtung (20, 30) im Verhältnis zur Matrize (10').

2. Verfahren nach Anspruch 1, wobei der Schritt des Herstellens der Positionierungsschiene (30) durch Thermoformen einen Schritt des Thermoformens einer Folie aus einem Material umfasst, das Neopren oder ein Elastomer umfasst und eine durchschnittliche Dicke zwischen 1,5 mm und 2,5 mm und vorzugsweise zwischen 1,7 mm und 2,3 mm aufweist.

3. Verfahren nach einem der Ansprüche 1 oder 2, umfassend einen Schritt des Modellierens mindestens eines Stifts (21) an dem Retainer (20) und wobei der Schritt des Herstellens des Retainers (20) mindestens einen Schritt des Herstellens des mindestens einen Stifts (21) umfasst.

4. Verfahren nach einem der Ansprüche 1 bis 3, umfassend einen Schritt des Modellierens mindestens eines Verbindungsstücks (22) an dem Retainer (20) und wobei der Schritt des Herstellens des Retainers (20) mit dem mindestens einen Verbindungsstück (22) einen Schritt des maschinellen Bearbeitens derselben Scheibe aus dem Material (40) umfasst.

5. Verfahren nach einem der Ansprüche 1 bis 4, ferner umfassend einen Schritt des Polierens und/oder einen Schritt des Reinigens des Retainers (20).

6. Verfahren nach einem der Ansprüche 1 bis 5, wobei die Scheibe (40) aus polymerem Verbundmaterial ein amorphes, vernetztes polymeres Verbundmaterial umfasst.

7. Verfahren nach einem der Ansprüche 1 bis 6, ferner umfassend einen Schritt des Abdrucknehmens von mindestens einem Teil eines Zahnbogens (10), der durch die Matrize (10') dargestellt werden soll.

8. Verfahren nach Anspruch 7, wobei der Schritt des Abdrucknehmens einen Schritt des intraoralen Scannens umfasst.

## Claims

1. Method for constructing a contention device (20, 30) including at least the following steps:
- a step of modelling a contention apparatus (20) in the form of a tape;
- a step of constructing the contention apparatus (20) by machining a slab (40) of composite polymer material or by 3D printing;
- a step of constructing a positioning duct (30) by thermoforming on a support assembly which includes the contention apparatus (20) and a die (10'), representing at least one dental arch (10), whereon the contention apparatus (20) is positioned;
- a step of obtaining the contention device (20, 30) on the die (10'), the contention device including the positioning duct (30) and the contention apparatus (20) in the form of a tape positioned in the positioning duct (30); and
- a step of separating the contention device (20, 30) from the die (10').

2. Method according to claim 1 wherein the step of constructing the positioning duct (30) by thermoforming includes a step of thermoforming a film of material including neoprene or an elastomer, and with a mean thickness between 1.5 mm and 2.5 mm, and preferably between 1.7 mm and 2.3 mm.

3. Method according to any one of claims 1 or 2 including a step of modelling at least one lug (21) on the contention apparatus (20) and wherein the step of constructing the contention apparatus (20) includes at least one step of constructing the at least one lug (21).

4. Method according to any one of claims 1 to 3 including a step of modelling at least one connector (22) on the contention apparatus (20) and wherein the step of constructing the contention apparatus (20) with the at least one connector (22) includes a step of machining the same slab of said material (40).

5. Method according to any one of claims 1 to 4 further including a step of polishing and/or a step of cleaning the contention apparatus (20).

6. Method according to any one of claims 1 to 5 wherein the slab (40) of composite polymer material includes a crosslinking amorphous composite polymer.

7. Method according to any one of claims 1 to 6 further including a step of taking an impression of at least the part of a dental arch (10) to be represented by the die (10').

8. Method according to claim 7 wherein the step of taking an impression includes an intraoral scanning step.
